# EUROPEAN PATENT SPECIFICATION

(11) **EP 4 205 737 B1**
(45) Date of publication and mention of the grant of the patent: **23.09.2026**
(21) Application number: 21854411.2
(22) Date of filing: 05.08.2021
(51) Int. Cl.: A61K 31/352, A61K 36/484, A61P 11/00, A61P 37/00, A61P 29/00, C07D 311/36

(54) **COMPOSITION FOR PREVENTION, AMELIORATION, OR TREATMENT OF INFLAMMATORY DISEASES COMPRISING GANCAONIN N AS ACTIVE INGREDIENT**
ZUSAMMENSETZUNG ZUR PRÄVENTION, LINDERUNG ODER BEHANDLUNG VON ENTZÜNDLICHEN ERKRANKUNGEN MIT GANCAONIN N ALS WIRKSTOFF
COMPOSITION POUR LA PRÉVENTION, L'AMÉLIORATION OU LE TRAITEMENT DE MALADIES INFLAMMATOIRES COMPRENANT LA GANCAONINE N EN TANT QUE PRINCIPE ACTIF

(30) Priority: 05.08.2020 KR 20200097800
(43) Date of publication of application: 05.07.2023
(73) Proprietor: UNIVERSITY-INDUSTRY COOPERATION GROUP OF KYUNG HEE UNIVERSITY, Yongin-si, Gyeonggi-do 17104 (KR)
(72) Inventor: JANG, Hyeung Jin, Seoul 06275 (KR)
(74) Representative: Ostertag & Partner Patentanwälte mbB
(86) International application number: PCT/KR2021/010367
(87) International publication number: WO 2022/031092

(56) References cited:
- WO-A1-2009/129372
- JP-A- 2015 093 848
- KR-A- 20050 035 142
- KR-A- 20090 036 676
- KR-A- 20190 118 919
- CHENG CAI-HONG ET AL: "A New Isoflavonoid from the Rhizomes of Cyperus rotundus", vol. 26, no. 13, 1 January 2014 (2014-01-01), IN, pages 3967 - 3970, XP093089624, ISSN: 0970-7077, Retrieved from the Internet <URL:http://dx.doi.org/10.14233/ajchem.2014.16131> DOI: 10.14233/ajchem.2014.16131
- KO HYUN MIN ET AL: "Gancaonin N from Glycyrrhiza uralensis Attenuates the Inflammatory Response by Downregulating the NF-[kappa]B/MAPK Pathway on an Acute Pneumonia In Vitro Model", vol. 13, no. 7, 6 July 2021 (2021-07-06), Switzerland, pages 1028, XP093175710, ISSN: 1999-4923, Retrieved from the Internet <URL:https://www.mdpi.com/1999-4923/13/7/1028/pdf> DOI: 10.3390/pharmaceutics13071028
- RIZZATO GIOVANNI ET AL: "A new exploration of licorice metabolome", FOOD CHEMISTRY, ELSEVIER LTD, NL, vol. 221, 17 November 2016 (2016-11-17), pages 959 - 968, XP029844800, ISSN: 0308-8146, DOI: 10.1016/J.FOODCHEM.2016.11.068
- LIU YAN-PING, GUO JIA-MING, YAN GUI, ZHANG MING-MING, ZHANG WEN-HAO, QIANG LEI, FU YAN-HUI: "Anti-Inflammatory and Antiproliferative Prenylated Isoflavone Derivatives from the Fruits of Ficus carica", JOURNAL OF AGRICULTURAL AND FOOD CHEMISTRY, AMERICAN CHEMICAL SOCIETY, US, vol. 67, no. 17, 1 May 2019 (2019-05-01), US , pages 4817 - 4823, XP055895420, ISSN: 0021-8561, DOI: 10.1021/acs.jafc.9b00865

## Description

### Technical Field

The present disclosure relates to a composition for the prevention, amelioration, or treatment of pneumonia, the composition including as an active ingredient gancaonin N, which is a flavonoid component derived from the root and aerial part of licorice (Glycyrrhiza aspera Pall).

### Background Art

Inflammation response is a body defense mechanism against various stimuli, and chronic inflammatory response can cause various diseases. Nitric oxide (NO) which is excessively produced by inducible nitric oxide synthase (iNOS) is involved in various inflammatory diseases, and effective iNOS blockers have value as therapeutic agents. When a living body is infected with a pathogen or when the immune system is hyperactive for some reason, macrophages start to act. Macrophages play a significant role in defending the host against pathogens with the help of pathogens and substances activating T cells, which are immune cells. This activity is regulated by several intermediates. Among them, nitrogen monoxide (NO) produced by macrophages acts as an important mediator to kill bacteria, fungi, and parasitic bacteria or to inhibit the growth of them. Nitric oxide (NO), an extremely unstable gas, is known to be an important macrophage mediator that kills pathogens and tumor cells or regulates the immune system in the human body. However, excessive generation of nitric oxide has side effects of killing host cells and causing inflammation. In the case where macrophages continue to excessively generate nitric oxide even after the death of the pathogens, it results in shock after sepsis or in severe inflammatory diseases, leading to an autoimmune disease. In other words, the insufficient generation of nitric oxide causes a problem in the role of macrophages as a mediator, but the excessive generation of nitric oxide causes inflammation or side effects. Therefore, the amount of nitric oxide produced by iNOS must be appropriately controlled. In addition, cyclo oxygenase-2 (COX-2) is an enzyme whose expression is induced by various inflammatory substances and cytokines. It has been found that COX-2 is abnormally excessively expressed in diverse types of cancer, and it has been reported that prostaglandin E2 (PGE2) produced by COX-2 is directly related to inflammatory response.

On the other hand, in recent years, interest in herbal medicines has increased in consideration of in vivo safety and the like. Therefore, many studies are being conducted on therapeutic agents using ingredients extracted from plants as active ingredients, and it is known that herbal medicines contain active ingredients that we have not yet recognized. Therefore, extracting effective pharmaceutical ingredients from herbal medicines has emerged as a new subject in research in modern pharmacology.

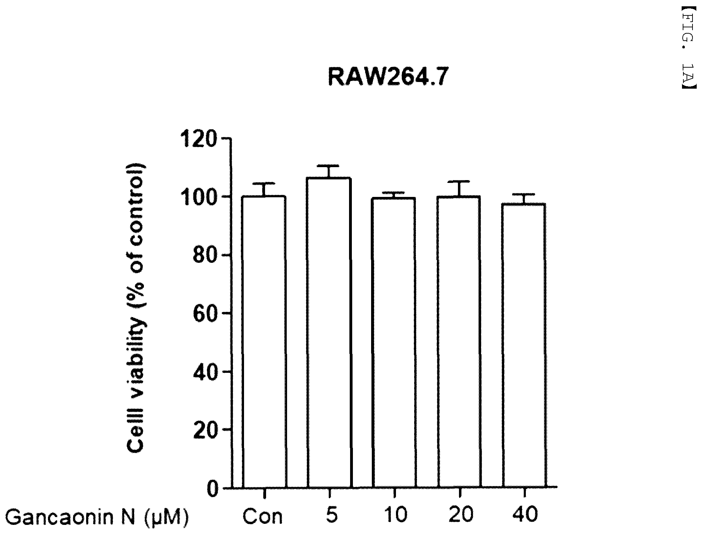

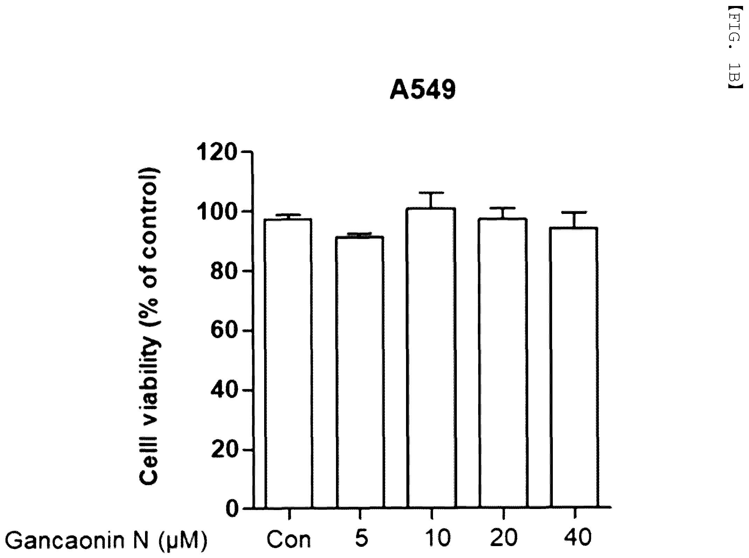

Liu Yan-Ping et al("Anti-Inflammatory and Antiproliferative Prenylated Isoflavone Derivatives from the Fruits of Ficus carica", Journal of Agricultural and Food Chemistry, American Chemical Society, US, vol. 67, no. 17, 1 May 2019 (2019-05-01), pages 4817-4823) discloses gancaonin N (i.e. compound 14 in figure 1) for use in treating inflammatory diseases through the inhibition of NO production induced by LPS in mouse macrophage RAW264.7 cells, inhibition which is comparable to the one of the positive control hydrocortisone.

### Disclosure

### Technical Problem

The present invention is defined in the appended claims. Embodiments not falling within the scope of the appended claims do not form part of the invention. Any references in the description to methods of treatment refer to the compounds, pharmaceutical compositions and medicaments of the present invention for use in a method for treatment of the human (or animal) body by therapy.

Under the above-described background, the inventors studied herbal ingredients effective for the prevention, amelioration, or treatment of inflammatory diseases and immunological diseases. As a result, it has been found that gancaonin N, which is a flavonoid component derived from the root and aerial part of licorice, inhibits the production of nitric oxide (NO), which is a key factor of inducing inflammation, inhibits the expression of iNOS and COX-2, which are known as inflammation-inducing factors, and has low cytotoxicity. On the basis of the findings, the inventors have completed the present disclosure.

Accordingly, an objective of the present disclosure is to provide a pharmaceutical composition for preventing or treating pneumonia, the composition including gancaonin N as an active ingredient.

Another objective of the present disclosure is to provide a health functional food composition for preventing or ameliorating pneumonia, the composition including gancaonin N as an active ingredient.

### Technical Solution

In order to accomplish the objectives, in one aspect of the present disclosure, there is provided a pharmaceutical composition for preventing or treating pneumonia, the composition including gancaonin N as an active ingredient.

In one embodiment of the present disclosure, the composition is capable of inhibiting the production of at least one selected from the group consisting of nitric oxide (NO), prostaglandin 2 (PGE2), and cyclo oxygenase-2 (COX-2).

In another embodiment of the present disclosure, the composition is capable of inhibiting the expression of pro-inflammatory cytokines and the expression of genes encoding the pro-inflammatory cytokines.

In a further embodiment of the present disclosure, the pro-inflammatory cytokine is any one or more selected from the group consisting of tumor necrosis factor-α (TNF-α), interleukin-1β (IL-1β), and interleukin 6 (IL-6).

In a yet further embodiment of the present disclosure, the composition is capable of inhibiting the expression of at least one selected from the group consisting of extracellular signal-regulated kinase (ERK), p38, and p65.

In a yet further embodiment of the present disclosure, the gancaonin N may be included in an amount of 5 to 60 µM.

In addition, in another aspect of the present disclosure, there is provided a health functional food composition for preventing or ameliorating pneumonia, the composition including gancaonin N as an active ingredient.

In a further aspect of the present disclosure, there is provided a method for preventing or treating pneumonia, the method including administering to a subject the pharmaceutical composition including gancaonin N as an active ingredient.

In a further aspect of the present disclosure, there is provided a use of the pharmaceutical composition in preventing or treating pneumonia.

### Advantageous Effects

According to the present disclosure, gancaonin N, which is a flavonoid component derived from the roots and aerial parts of licorice (Glycyrrhiza aspera Pall), is confirmed to inhibit the production of nitric oxide (NO), which is a key factor of inducing inflammation, to inhibit the expression of PGE2, iNOS and COX-2, which are known as factors inducing inflammation, and not to exhibit cytotoxicity. Therefore, the composition containing gancaonin N as an active ingredient, which is proposed by the present disclosure, can be usefully used as a safe natural therapeutic agent with few side effects for inflammatory diseases and more specifically for lung inflammation.

### Description of Drawings

FIG. 1A shows results of measuring cell viability for RAW264.7 cells treated with various concentrations (5, 10, 20, 40 µM) of gancaonin N and cultured for 24 hours;
FIG. 1B shows results of measuring cell viability for A549 cells treated with various concentrations (5, 10, 20, 40 µM) of gancaonin N and cultured for 24 hours;

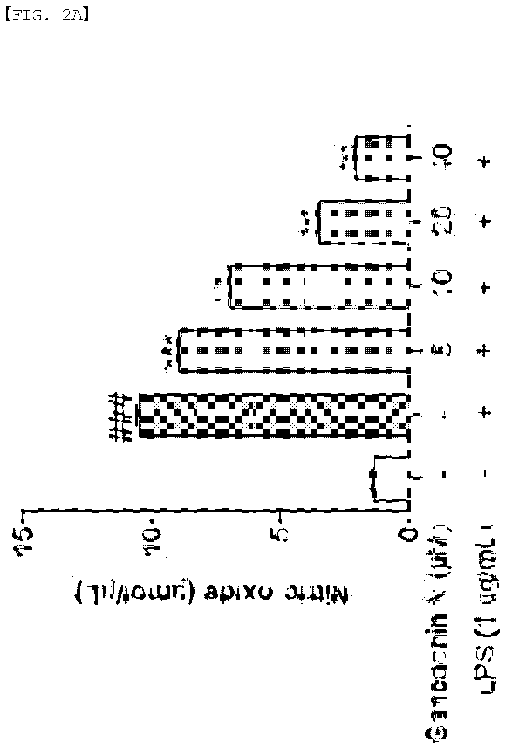

FIG. 2A shows results of measuring change in the production of nitric oxide (NO) for RAW264.7 cells stimulated with LPS and treated with gancaonin N according to concentrations (0, 5, 10, 20, 40 µM) of gancaonin N (in which "###P < 0.001" indicates significance compared to a negative control group, and each of "*p < 0.05", "**p < 0.01", and "***p < 0.001" indicates significance compared to an LPS-only treatment group);

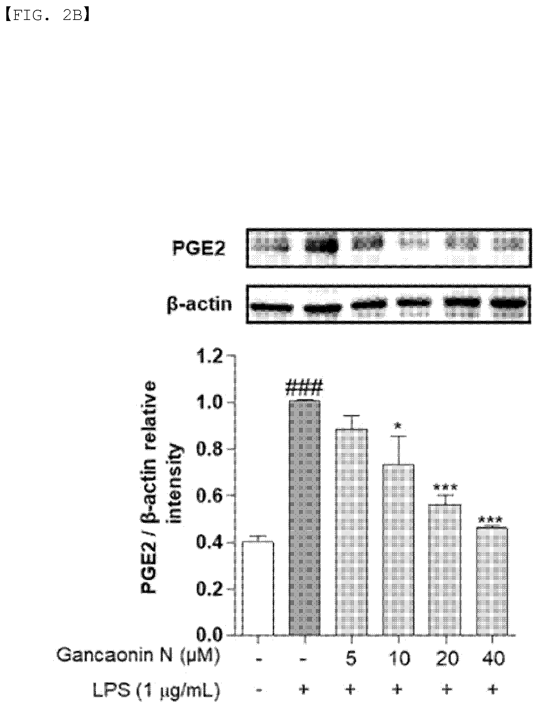

FIG. 2B shows results of measuring change in the production of prostaglandin E2 (PGE2) protein for RAW264.7 cells stimulated with LPS and treated with gancaonin N according to concentrations (0, 5, 10, 20, 40 µM) of Gancaonin N (in which "###P < 0.001" indicates significance compared to a negative control group, and each of "*p < 0.05", "**p < 0.01", and "***p < 0.001" indicates significance compared to an LPS-only treatment group);

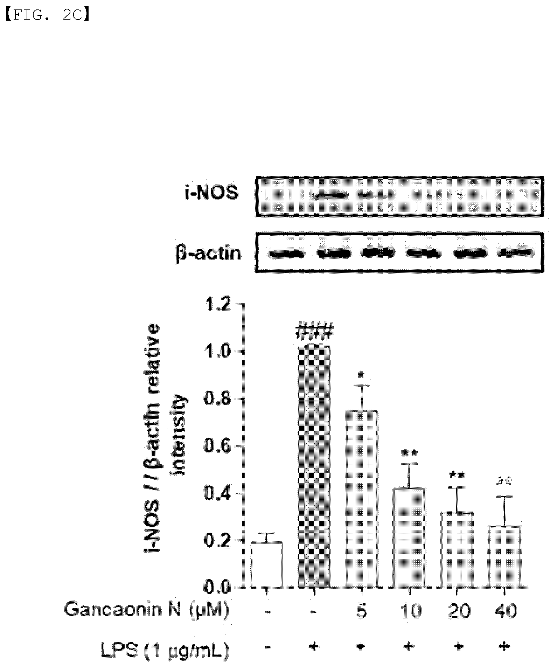

FIG. 2C shows results of measuring change in the production of nitric oxide synthase (iNOS) for RAW264.7 cells stimulated with LPS and treated with gancaonin N according to concentrations (0, 5, 10, 20, 40 µM) of gancaonin N (in which "###P < 0.001" indicates significance compared to a negative control group, and each of "*p < 0.05", "**p < 0.01", and "***p < 0.001" indicates significance compared to an LPS-only treatment group);

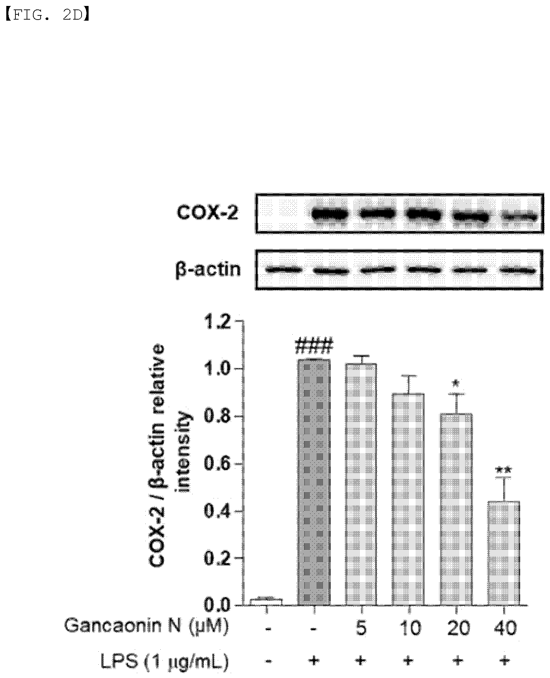

FIG. 2D shows results of measuring change in the production of cyclo oxygenase-2 (COX-2) for RAW264.7 cells stimulated with LPS and treated with gancaonin N according to concentrations (0, 5, 10, 20, 40 µM) of gancaonin N (in which "###P < 0.001" indicates significance compared to a negative control group, and each of "*p < 0.05", "**p < 0.01", and "***p < 0.001" indicates significance compared to an LPS-only treatment group);

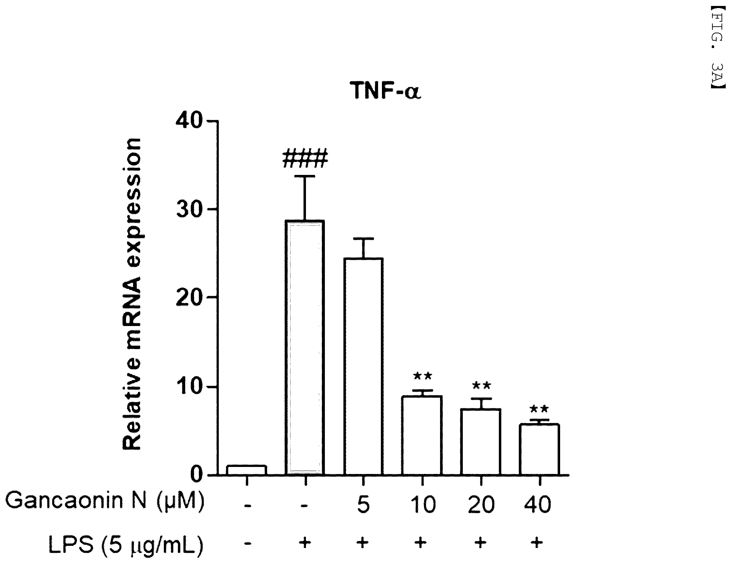

FIG. 3A shows results of measuring change in the mRNA expression level of tumor necrosis factor-a (TNF-α), which is a pro-inflammatory cytokine, for A549 cells stimulated with LPS and treated with gancaonin N according to concentrations (0, 5, 10, 20, 40 µM) of gancaonin N (in which "###P < 0.001" indicates significance compared to a negative control group, and each of "*p < 0.05", "**p < 0.01", and "***p < 0.001" indicates significance compared to an LPS-only treatment group);

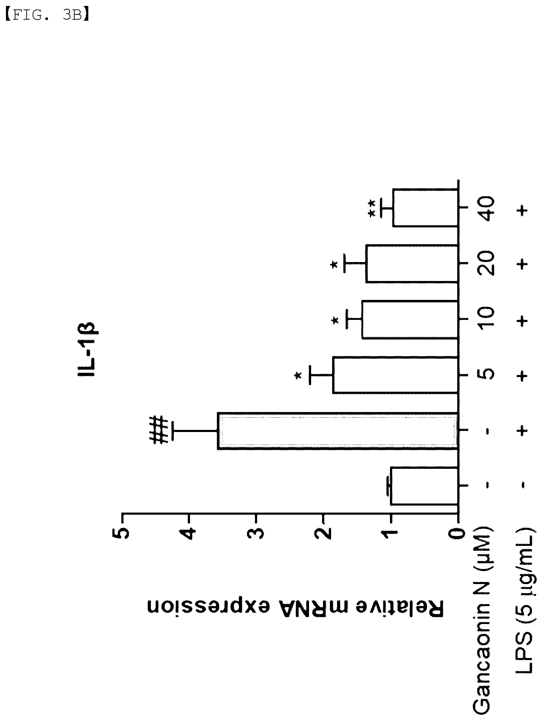

FIG. 3B shows results of measuring change in the mRNA expression level of interleukin-1β (IL-1β), which is a pro-inflammatory cytokine, for A549 cells stimulated with LPS and treated with gancaonin N according to concentrations (0, 5, 10, 20, 40 µM) of gancaonin N (in which "###P < 0.001" indicates significance compared to a negative control group, and each of "*p < 0.05", "**p < 0.01", and "***p < 0.001" indicates significance compared to an LPS-only treatment group);

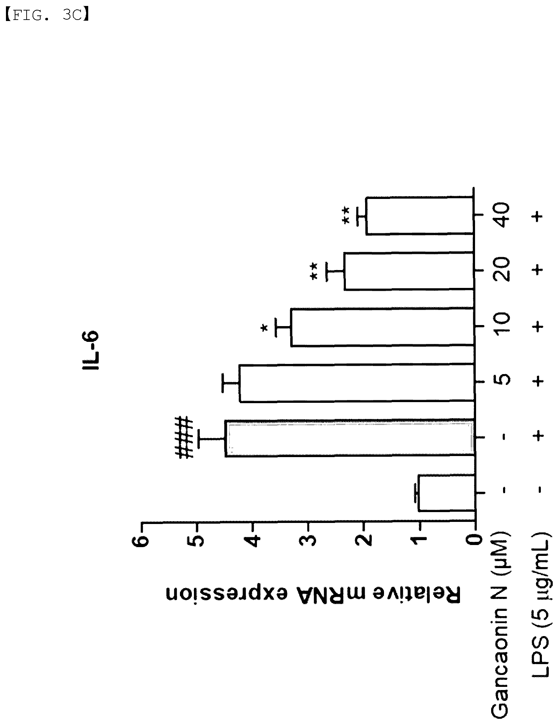

FIG. 3C shows results of measuring change in the mRNA expression level of interleukin-6 (IL-6), which is a pro-inflammatory cytokine, for A549 cells stimulated with LPS and treated with gancaonin N according to concentrations (0, 5, 10, 20, 40 µM) of gancaonin N (in which "###P < 0.001" indicates significance compared to a negative control group, and each of "*p < 0.05", "**p < 0.01", and "***p < 0.001" indicates significance compared to an LPS-only treatment group);

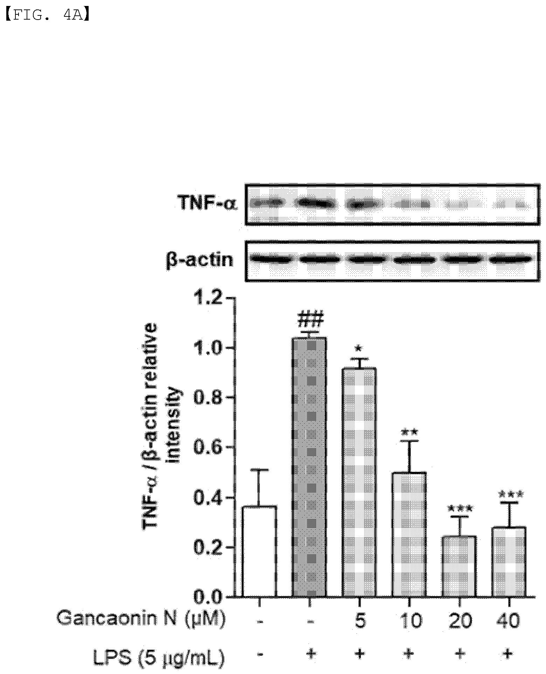

FIG. 4A shows results of measuring change in the protein expression level of tumor necrosis factor-a (TNF-α), which is a pro-inflammatory cytokine, for A549 cells stimulated with LPS and treated with gancaonin N according to concentrations (0, 5, 10, 20, 40 µM) of gancaonin N used to treat (in which "###P < 0.001" indicates significance compared to a negative control group, and each of "*p < 0.05", "**p < 0.01", and "***p < 0.001" indicates significance compared to an LPS-only treatment group);

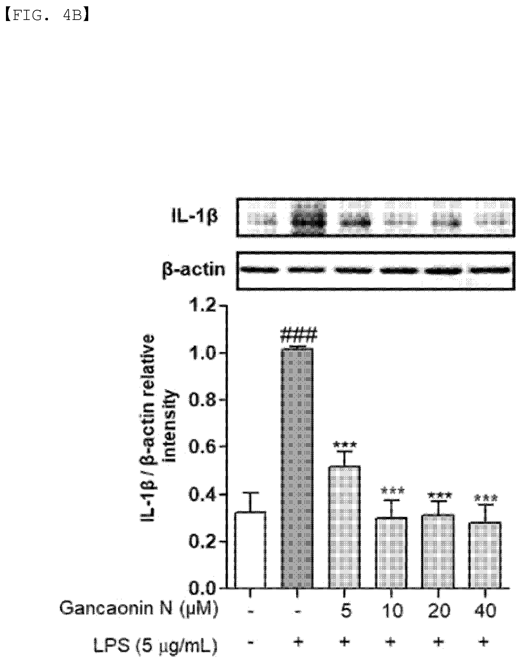

FIG. 4B shows results of measuring change in the protein expression level of interleukin-1β (IL-1β), which is a pro-inflammatory cytokine, for A549 cells stimulated with LPS and treated with gancaonin N according to concentrations (0, 5, 10, 20, 40 µM) of gancaonin N used to treat (in which "###P < 0.001" indicates significance compared to a negative control group, and each of "*p < 0.05", "**p < 0.01", and "***p < 0.001" indicates significance compared to an LPS-only treatment group);

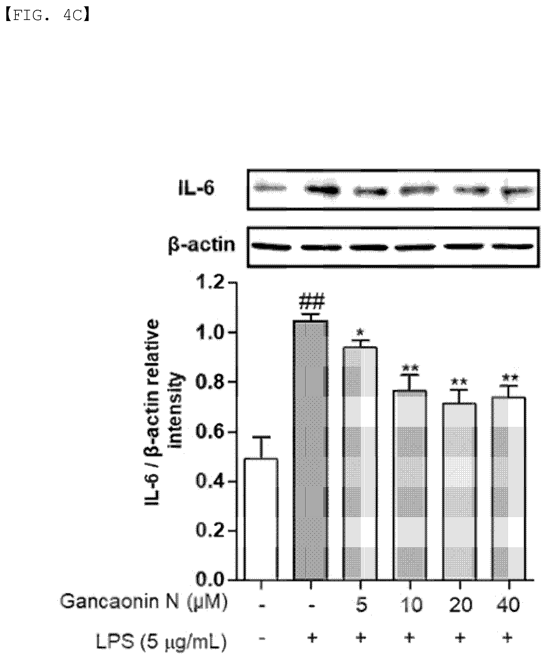

FIG. 4C shows results of measuring change in the protein expression level of interleukin-6 (IL-6), which is a pro-inflammatory cytokine, for A549 cells stimulated with LPS and treated with gancaonin N according to concentrations (0, 5, 10, 20, 40 µM) of gancaonin N used to treat (in which "###P < 0.001" indicates significance compared to a negative control group, and each of "*p < 0.05", "**p < 0.01", and "***p < 0.001" indicates significance compared to an LPS-only treatment group);

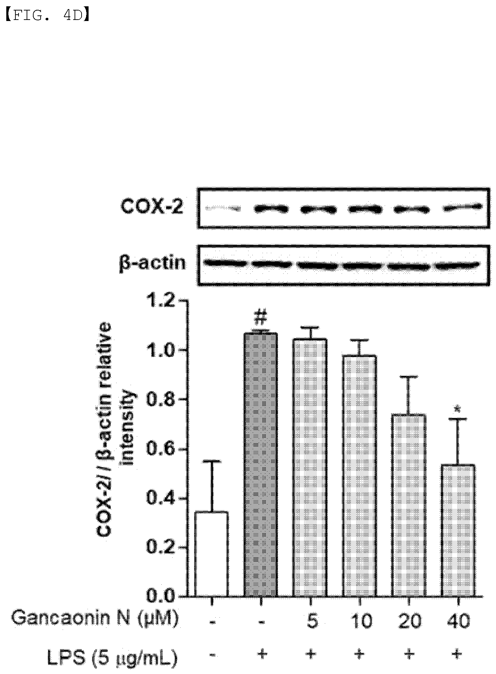

FIG. 4D shows results of measuring change in the protein level of cyclo oxygenase-2 (COX-2) for A549 cells stimulated with LPS and treated with gancaonin N according to concentrations (0, 5, 10, 20, 40 µM) of gancaonin N used to treat (in which "###P < 0.001" indicates significance compared to a negative control group, and each of "*p < 0.05", "**p < 0.01", and "***p < 0.001" indicates significance compared to an LPS-only treatment group);
FIG. 4C shows results of investigation of the protein level of extracellular signal-regulated kinase (ERK) and the activity level thereof in A549 cells stimulated with LPS and treated with gancaonin N, according to concentrations (0, 5, 10, 20, 40 µM) of gancaonin N used to treat the A549, the investigation being performed to examine the effect of gancaonin N on a MAPK/NF-kB signaling pathway (in which "###P < 0.001" indicates significance compared to a negative control group, and each of "*p < 0.05", "**p < 0.01", and "***p < 0.001" indicates significance compared to an LPS-only treatment group);

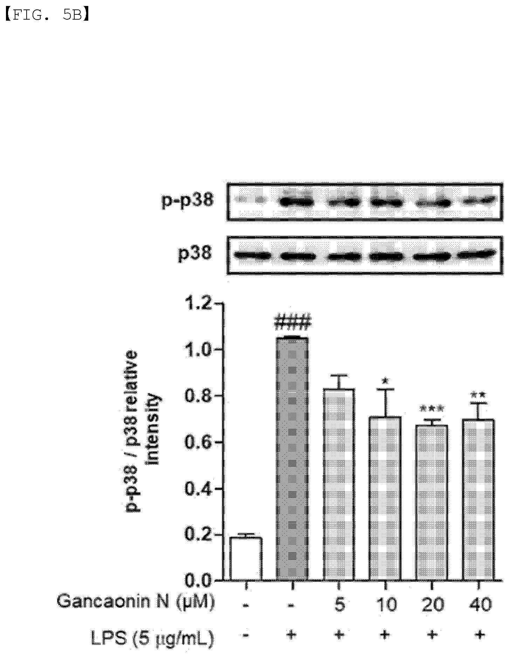

FIG. 5B shows results of investigation of the protein level of p-38 and the activity level thereof in A549 cells stimulated with LPS and treated with gancaonin N, according to concentrations (0, 5, 10, 20, 40 µM) of gancaonin N used to treat the A549, the investigation being performed to examine the effect of gancaonin N on a MAPK/NF-kB signaling pathway (in which "###P < 0.001" indicates significance compared to a negative control group, and each of "*p < 0.05", "**p < 0.01", and "***p < 0.001" indicates significance compared to an LPS-only treatment group);

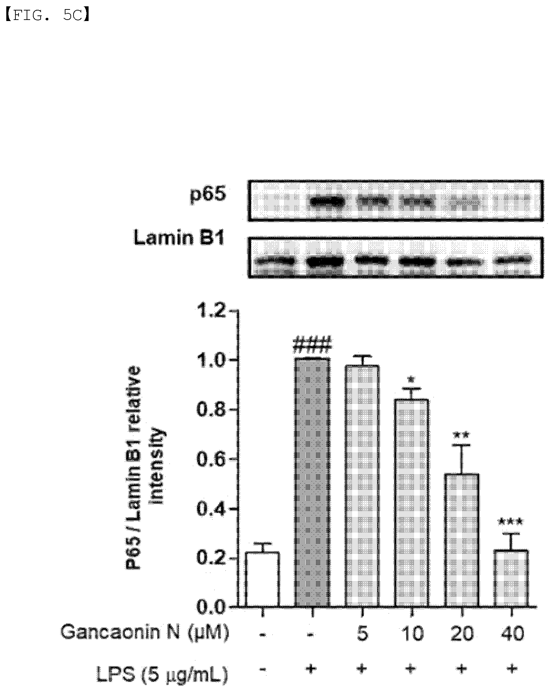

FIG. 5C shows results of investigation of the protein level of p65 and the activity level thereof in A549 cells stimulated with LPS and treated with gancaonin N according to concentrations (0, 5, 10, 20, 40 µM) of gancaonin N used to treat the A549, the investigation being performed to examine the effect of gancaonin N on a MAPK/NF-kB signaling pathway (in which "###P < 0.001" indicates significance compared to a negative control group, and each of "*p < 0.05", "**p < 0.01", and "***p < 0.001" indicates significance compared to an only-LPS treatment group);

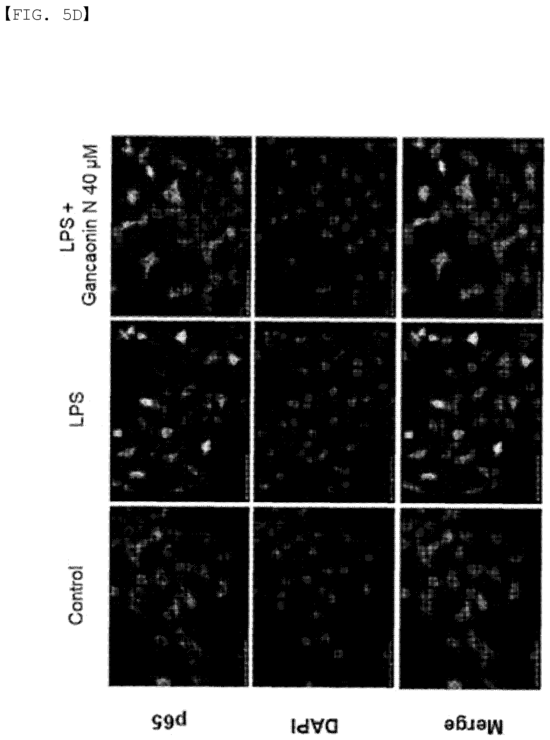

FIG. 5D shows results of investigating the expression of NF-κB p65 for A549 cells stimulated with LPS and stained through immunofluorescence staining in order to examine the effect of gancaonin N on the MAPK/NF-κB signaling pathway;

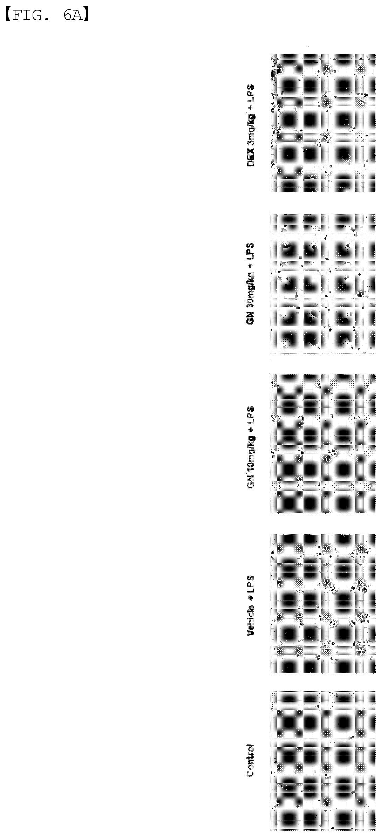

FIG. 6A shows results of treating a control group and an LPS-induced acute lung injury (ALI) mouse model with 10 mg/kg of gancaonin N (GN 10 mg/kg), 30 mg/kg of gancaonin (GN 30 mg/kg), or 3 mg/kg of dexamethasone (DEX 3 mg/kg) having an anti-inflammatory effect, followed by staining with a Diff-Quik kit;

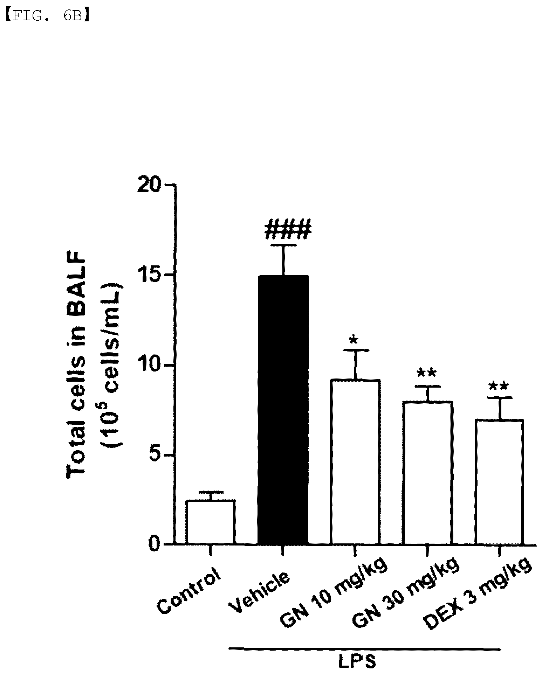

FIG. 6b shows results of counting the total number of cells in Bronchoalveolar lavage fluid (BALF) while administering 10 mg/kg of gancaonin N, 30 mg/kg of gancaonin, or 3 mg/kg of dexamethasone having an anti-inflammatory effect to a control group and an LPS-induced acute lung injury (ALI) mouse model;

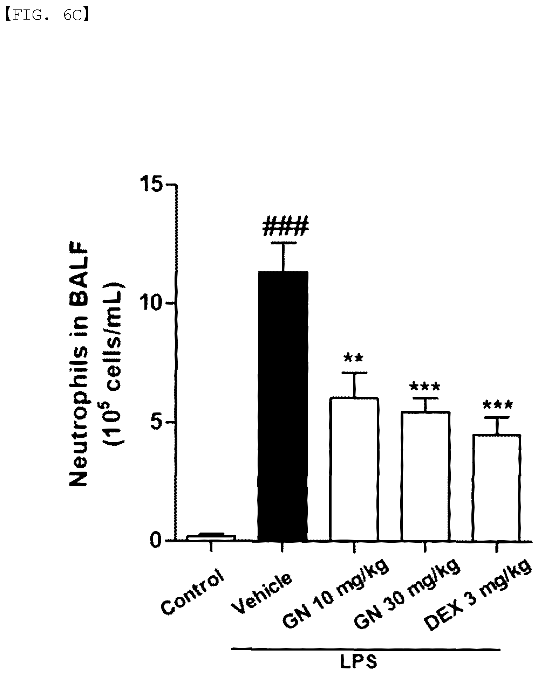

FIG. 6C shows results of counting the number of neutrophils in bronchoalveolar lavage fluid (BALF) while administering 10 mg/kg of gancaonin N, 30 mg/kg of gancaonin, or 3 mg/kg of dexamethasone having an anti-inflammatory effect to a control group and an LPS-induced acute lung injury (ALI) mouse model;

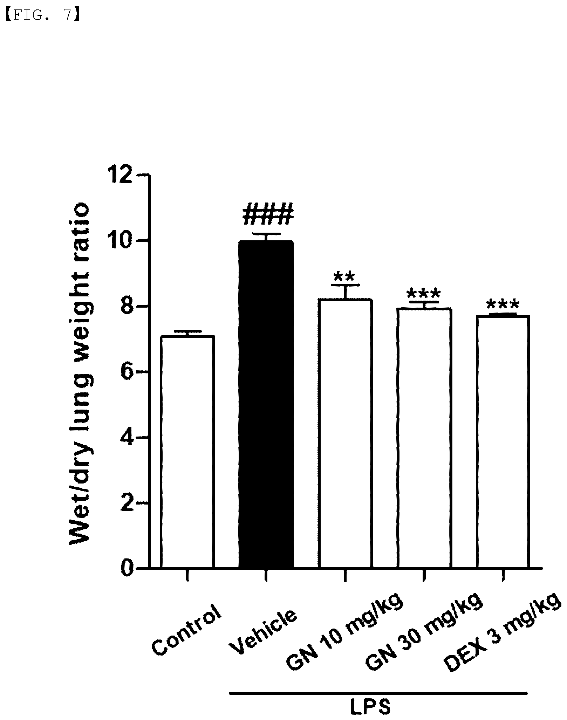

FIG. 7 shows results of investigating lung wet-to-dry (W/D) ratios for a control group and an LPS-induced acute lung injury (ALI) mouse model while administering 10 mg/kg of gancaonin N, 30 mg/kg of gancaonin, or 3 mg/kg of dexamethasone having an anti-inflammatory effect to the control group and the LPS-induced acute lung injury (ALI) mouse model; and

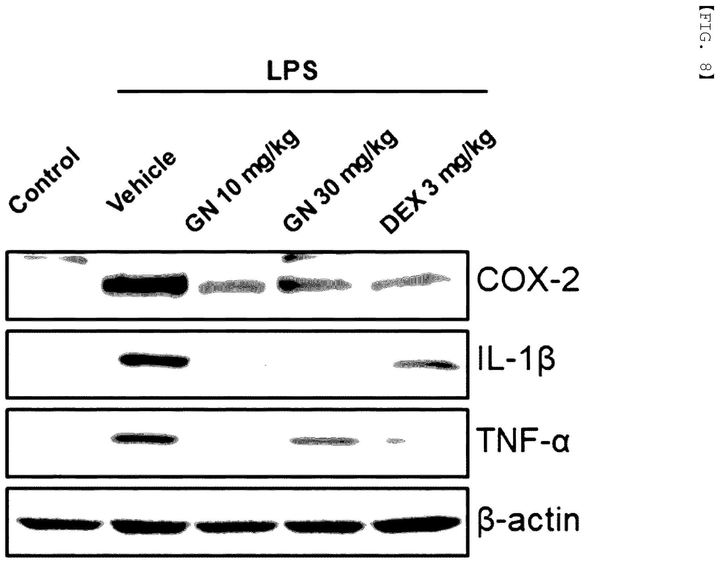

FIG. 8 shows results of investigating the levels of inflammatory proteins for a control group and an LPS-induced acute lung injury (ALI) mouse model while administering 10 mg/kg of gancaonin N, 30 mg/kg of gancaonin, or 3 mg/kg of dexamethasone having an anti-inflammatory effect to the control group and the LPS-induced acute lung injury (ALI) mouse model.

### Best Mode

The inventors studied herbal ingredients effective for the prevention, amelioration, or treatment of inflammatory diseases and immunological diseases. As a result, it has been found that gancaonin N, which is a flavonoid component derived from the root and aerial part of licorice, inhibits the production of nitric oxide (NO), which is a key factor of inducing inflammation, inhibits the expression of iNOS and COX-2, which are known as inflammation-inducing factors, and has low cytotoxicity. On the basis of the findings, the inventors have completed the present disclosure.

Thus, in one aspect of the present disclosure, there is provided a pharmaceutical composition including gancaonin N as an active ingredient for preventing or treating pneumonia.

In the present disclosure, the gancaonin N is one of the flavonoid components derived from the root and aerial parts of licorice. The IUPAC name of gancaonin N is 5,7-dihydroxy-3-(2-hydroxy-4-methoxyphenyl)-6-(3-methylbut-2-enyl)chromen-4-one, the molecular formula is C₂₁H₂₀O₆, and the chemical structure is as shown below.

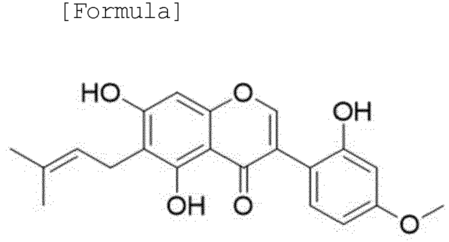

The composition may inhibit the expression of nitric oxide (NO), prostaglandin 2 (PGE2) or cyclo oxygenase-2 (COX-2).

The term "nitric oxide (NO)" used herein is one of the representative inflammation-related factors that play a role in vasodilation. When macrophages are stimulated, nitric oxide (NO) is produced in the process in which inducible nitric oxide synthase (iNOS), which is an enzyme, converts L-arginine into L-citrulline. That is, NO is produced by the stimulated macrophages.

The term "prostaglandin 2 (PGE2)" used herein is one of the eicosanoids biosynthesized from arachidonic acid that can act as a mediator of an inflammatory response and is synthesized by the action of COX-2.

The pharmaceutical composition of the present disclosure may be a composition capable of inhibiting the expression of pro-inflammatory cytokines and the expression of genes encoding the pro-inflammatory cytokines. The pro-inflammatory cytokine may be tumor necrosis factor-α (TNF-α), interleukin-1 beta (IL-1β), or interleukin 6 (IL-6), but is not limited thereto. In addition, the pharmaceutical composition of the present disclosure may inhibit the expression of extracellular signal-regulated kinase (ERK), p38, or p65, meaning that the gancaonin N-containing composition of the present disclosure can regulate a MAPK/NF-κB signaling pathway associated with inflammatory diseases.

The pharmaceutical composition may include gancaonin N in an amount of 5 to 60 µM, preferably 5 to 50 µM, more preferably 5 to 40 µM, but the amount is not limited thereto.

The term "prevention" used herein refers to any action that suppresses or delays the onset pneumonia by administration of the pharmaceutical composition of the present disclosure.

As used herein, the term "treatment" refers to all activities in which symptoms of pneumonia are ameliorated or beneficially changed by administration of the pharmaceutical composition of the present disclosure.

Through examples of the present disclosure, the inventors have found that when Raw264.7 cells or A549 cells were treated with the gancaonin N-containing composition of the present disclosure, the composition containing gancaonin N did not show no cytotoxicity (Example 2) and significantly inhibited the expression of nitric oxide (Example 3), the expression of inflammation-related factors (Example 4), and the expression of pro-inflammatory factors (Example 5).

In another example, when a mouse model in which acute lung injury is induced by LPS is treated with gancaonin N, the inventors have found that inflammatory cell infiltration was reduced, the severity of pulmonary edema was alleviated, and the expression of pro-inflammatory factor protein was reduced. The inventors also have confirmed that the effects were at a level similar to that of dexamethasone which had been previously used as an anti-inflammatory agent (Example 7).

On the basis of the results described above, the inventors specifically have confirmed that gancaonin N was capable of significantly inhibiting inflammation without causing cytotoxicity.

The pharmaceutical composition according to the present disclosure includes Gancaonin N as an active ingredient and may further include a pharmaceutically acceptable carrier. The pharmaceutically acceptable carrier is a carrier commonly used in formulations, and examples thereof include, but are not limited to, saline, sterile water, Ringer's solution, buffered saline, cyclodextrin, dextrose solution, maltodextrin solution, glycerol, ethanol, liposome, and the like. If necessary, other conventional additives such as antioxidants and buffers may be added thereto. In addition, diluents, dispersants, surfactants, binders, lubricants, and the like may be additionally added to make the composition of the present disclosure as injectable formulations such as an aqueous solution, suspension, and emulsion, or make the composition of the present disclosure as pills, capsules, granules, or tablets. Regarding suitable pharmaceutically acceptable carriers and formulations, formulations can be preferably made according to each component using the method disclosed in Remington's literature. The pharmaceutical composition of the present disclosure is not particularly limited in formulations but may be preferably formulated as injections, inhalants, or skin preparations for external use.

The pharmaceutical composition of the present disclosure may be administered orally or administered parenterally (for example, intravenously, subcutaneously, intraperitoneally, or topically) as desired, and the dosage may vary depending on the condition and weight of the patient, the severity of disease, drug forms, the route and time of administration, and may be appropriately selected by those skilled in the art.

The composition according to the present disclosure is administered in a pharmaceutically effective dosage. In the present disclosure, the pharmaceutically effective dosage means an amount that is sufficient to treat or diagnose a disease with a reasonable benefit/risk ratio applicable to medical treatment, and the effective dosage depends on various factors including the type and severity of a disease, drug activity on the disease, patient sensitivity to drug, administration time, administration route, excretion rate, treatment period, and co-used drugs, and other factors well known in the medical field. The composition according to the present disclosure may be administered as an individual therapeutic agent or administered in combination with other therapeutic agents. When administered in combination, the composition and other therapeutic agents may be administered sequentially or simultaneously. The composition may be administered as a single dose or multiple doses. In consideration of all of the above factors, it is important to administer a dosage that can obtain the maximum efficacy with a minimum amount without side effects, and the dosage can be easily determined by those skilled in the art.

Specifically, the effective dosage of the pharmaceutical composition of the present disclosure may vary depending on the age, sex, condition, and body weight of the patient, the absorption rate, inactivation rate, and excretion rate of the active ingredient in the body, the type of disease, and concomitant drugs. Generally, the effective dosage may be in a range of 0.001 mg to 150 mg and preferably in a range of 0.01 mg to 100 mg per 1 kg of body weight, and the dosage may be administered daily or every other day, or divided into 1 to 3 doses a day. However, the dosage does not in any way limit the scope of the present disclosure because it can be increased or decreased depending on the route of administration, the severity of disease, the sex, weight, age, and the like of the patient, etc.

The inventors identified novel uses of gancaonin N for the prevention and treatment of pneumonia through specific experimental examples.

In another aspect of the present disclosure, there is provided a health functional food composition for preventing or ameliorating pneumonia, the composition including gancaonin N as an active ingredient.

As used herein, the term "amelioration" refers to any action that reduces at least one parameter related to a condition to be treated, for example, the degree of a symptom. In this case, the health functional food composition may be used simultaneously with or separately from a drug for treatment before or after the onset of the disease in order to prevent or ameliorate pneumonia.

The term "health function food composition" as used herein is a formulation selected from the group consisting of tablets, pills, powders, granules, powders, capsules, and liquid formulations, including one or more of carriers, diluents, excipients, and additives. Foods that can be added to the extract of the present disclosure include various foods, powders, granules, tablets, capsules, syrups, beverages, gum, tea, vitamin complexes, and health functional foods. Additives that can be added to the composition of the present disclosure include at least one selected from the group consisting of natural carbohydrates, taste modulators, nutrients, vitamins, minerals (electrolytes), flavors (synthetic flavors, natural flavors, etc.), colorants, fillers, pectic acid and salts thereof, alginic acid and salts thereof, organic acids, protective colloidal thickeners, pH adjusting agents, stabilizers, preservatives, antioxidants, glycerin, alcohol, carbonation agents, and the flesh of fruits. Examples of the natural carbohydrate include: monosaccharides such as glucose, fructose and the like; disaccharides such as maltose, sucrose, and the like; and polysaccharides such as conventional sugars (for example, dextrin and cyclodextrin) and sugar alcohols such as xylitol, sorbitol, and erythritol. As the flavors, natural flavors such as taumatin, stevia extract (for example, rebaudioside A, glycyrrhizin, etc.) and synthetic flavors such as saccharin, aspartame, etc. may be advantageously used. Aside from the ingredients described above, the composition of the present disclosure may further include various nutrients, vitamins, minerals (electrolytes), flavors such as synthetic flavors and natural flavors, colorants and color enhancers, pectic acid and salts thereof, alginic acid and salts thereof, organic acids, protective colloidal thickeners, pH adjusters, stabilizers, preservatives, glycerin, alcohols, carbonation agents used in carbonated beverages, and the like. In addition, the composition according to the present disclosure may contain fruit flesh for preparing natural fruit juice and vegetable beverages. These ingredients may be used solely or in combination. Specific examples of the carrier, excipient, diluent, and additive include, but are not limited to, at least one selected from the group consisting of lactose, dextrose, sucrose, sorbitol, mannitol, erythritol, starch, gum acacia, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methyl hydroxy benzoate, propylhydroxy benzoate, talc, magnesium stearate, and mineral oil.

In a further aspect of the present disclosure, there is provided a method of preventing or treating pneumonia, the method including administering to an individual the pharmaceutical composition described above.

Herein, the term "individual" means a subject in need of treatment of a disease and more specifically a mammal such as humans or non-human primates, mice, rats, dogs, cats, horses, cows, etc.

In a further aspect of the present disclosure, there is provided preventive and therapeutic uses of the pharmaceutical composition for pneumonia.

Hereinbelow, various examples will be described to aid in understanding the present disclosure. However, the examples described below are provided only to facilitate the understanding of the present disclosure and thus the details in the examples should not be construed to limit the scope of the present disclosure.

### Example 1. Raw Material Preparation and Experimental Method

### 1-1. Chemical Preparation

Gancaonin N, which is a flavonoid component derived from licorice root and aerial parts, was purchased from ChemFaces (in China). (3-(4,5-dimethyl-2-thiazolyl)-2,5-diphenyl-2[2H]-tetrazolium bromide (MTT) was purchased from Invitrogen (in Waltham, MA), and primary antibodies against COX-2, NF-κB p65, extracellular signal-regulated kinase (ERK), p-ERK, p38, p-p38, and Lamin B1 were purchased from Cell Signaling Technology (in Beverly, MA, USA). Primary antibodies against interleukin-6 (IL-6), IL-1β, and tumor necrosis factor-a (TNF-α) were from Proteintech (in Rosemont, IL, USA), and a primary antibody against PGE2 were purchased from Bioss (in Woburn, MA, USA), and a primary antibody against iNOS was purchased from R&D Systems Inc. (in Minneapolis, MN, USA). β-Actin primary antibody, anti-rabbit IgG-HRP, and anti-mouse IgG-HRP were purchased from Santa Cruz Biotechnology (in Dallas, TX, USA).

### 1-2. Preparation and Culturing of Test Cell Line

RAW264.7 murine macrophage cells were purchased from Korean Cell Line Bank (in Seoul, South Korea). A549 human alveolar basal epithelial cells were purchased from American Type Culture Collection (in ATCC, MD, USA). The cells were cultured in DMEM medium (manufactured by Corning Inc., NY, USA) containing 10% FBS (manufactured by Gibco, NY, USA) and 1X antibiotic-antimycotic solution (manufactured by ABAM, Corning Inc., NY, USA) under conditions of 37°C and 5% CO₂.

### 1-3. Preparation of Test Animal

25 6-week-old female BALB/c mice having a weight of 18g to 20 g were purchased from BioLink (DBL CO., Eumseong, Chungcheongbuk-do, Korea), and reared in the animal laboratory of KyungHee University. To establish an LPS-induced acute lung injury model, all the mice were divided into five groups each group of which consisted of 5 mice, and the five groups were referred to as control, vehicle (only LPS treatment), gancaonin N (10 mg/kg), gancaonin N (30 mg/kg), and dexamethasone (3 mg/kg) . Gancaonin N was intraperitoneally injected at a dose of 10 mg/kg or 30 mg/kg, and dexamethasone was intraperitoneally injected at a dose of 3 mg/kg as a positive control group. In addition, the control and vehicle groups were intraperitoneally injected with 4% DMSO in which gancaonin N was dissolved. Then, 10 µg/50 µL of LPS was intratracheally administered to the mice except for the control group one hour later (the control group mice were administered 50 µL of PBS), and the mice were sacrificed 12 hours later.

### 1-4. Cell Viability Analysis

To evaluate the cytotoxicity of gancaonin N, RAW264.7 cells and A549 cells were dispensed into 96-well plates (1 × 10⁴ cells/well) and cultured under conditions of 37°C and 5% CO₂. After the culturing, the cells were treated with gancaonin N at concentrations of 5, 10, 20, or 40 µM for 24 hours, and MTT solution (5 mg/mL) was added to each well. After adding 100 µL of dimethyl sulfoxide (DMSO, manufactured by Sigma-Aldrich, St. Louis, MO, USA), the absorbance was measured at 540 nm using a microplate reader (manufactured by Bio-Rad Hercules, CA, USA).

### 1-5. Nitric Oxide (NO) Measurement

Raw264.7 cells and A549 cells were dispensed into 6-well plates (6 × 10⁵ cells/well) and cultured under conditions of 37°C and 5% CO₂. After the culturing, the cells were treated with 1 µg/mL of lipopolysaccharide (LPS) and with 5 µM to 40 µM of gancaonin N and cultured for 24 hours, and the concentration of NO produced was determined using the cell culture supernatant. The production of NO was measured by reading the absorbance at 540 nm using Griess reagent. Then, a standard curve was drawn using sodium nitrate, and the result value was expressed as nitrate concentration.

### 1-6. Western Blotting Analysis

RAW264.7 cells and A549 cells were dispensed into 6-well plates (1 × 10⁶ cells/well) and cultured under conditions of 37°C and 5% CO₂. After the culturing, the Raw 264.7 cells were treated with 1 ug/mL of lipopolysaccharide (LPS) and the A549 cells were treated with 5 µg/mL of lipopolysaccharide (LPS) and with 5 µM to 40 µM of gancaonin N, and the cells were cultured for 24 hours or 6 hours. Then, the cells were lysed using a lysis buffer. In addition, the left lung extracted from each mouse was lysed using RIPA buffer. SDS-PAGE electrophoresis was performed on the cell and tissue lysates to separate proteins by side, and 5% bovine serum albumin was used for culturing and for blocking antibodies against PGE2 (1:500), IL-1β (1:500), IL-6 (1:500), TNF-α (1:500), i-NOS (1:500), COX-2 (1:1000), P-ERK1/2 (1:1000), ERK1/2 (1:3000), P-p38 (1:1000), p38 (1:3000), NF-κB p65 (1:1000), Lamin B1 (1:1000), and β-actin (1: 5,000).

### 1-7. Immunofluorescence Assay

A549 cells were dispensed into 6-well plates (1 × 10⁴ cells/well) and cultured under conditions of 37°C and 5% CO₂. After the culturing, the A549 cells were treated with of 5 µg/mL of lipopolysaccharide (LPS) and with 5 µM to 40 µM of gancaonin N, and the cells were then cultured for 6 hours. Thereafter, the cells were fixed with 10% formalin and treated with 0.2% Triton X for 20 minutes. Then, after blocking with 5% bovine serum, NF-κB p65 (1:300) antibody was incubated overnight at 4°C. After incubation along with Alexa-Fluor-488 antibody for one hour at room temperature, 4,6-diamidino-2-phenylindole (DAPI; Sigma-Aldrich, St. Louis, MO, USA) was used to stain the cell nuclei. The staining was performed at room temperature for 3 minutes. For fluorescence observation, EVOSR Cell Imaging systems (Thermo Fisher Scientific, Waltham, MA, USA) were used.

### 1-8. Real-Time PCR Analysis

A549 cells were dispensed into 6-well plates (1 × 10⁶ cells/well) and cultured under conditions of 37°C and 5% CO₂. After the culturing, the A549 cells were treated with of 5 µg/mL of lipopolysaccharide (LPS) and with 5 µM to 40 µM of gancaonin N, and the cells were then cultured for 24 hours. Afterwards, total RNA was isolated from each treated cell using Hybrid-RTM (GeneAll, South Korea). The isolated total RNA (500 ng) was converted to cDNA sequentially through incubation at 55°C for 60 minutes using oligo (dT) and incubation at 85°C for 5 minutes, and the cDNA was stored at 4°C until being used. In addition, real-time quantitative PCR was performed using Universal SYBR Green Master Mix (Applied Biosystems, USA), and the cDNA was amplified under conditions described below. 95°C for 15 minutes, then 95°C for 30 seconds, 59°C for 30 seconds, and 72°C for 30 seconds were repeated 40 times. Real-time PCR analysis was performed with the Applied Biosystems StepOne system (Applied Biosystems, USA), and the primer sequences used in the analysis are shown in Table 1 below.

**[Table 1]**

| | | |
|---|---|---|
| TNF-α | 5'-GCAGGTCTACTTTGGGTCATTG- 3'(Forward)(SEQ ID NO: 1) | 5'-GCGTTTGGGAAGGTTGGA-3' (Reverse) (SEQ ID NO: 2) |
| IL-1β | 5'-TCAGCCAATCTTCATTGCTCAA- 3'(Forward)(SEQ ID NO: 3) | 5'- TGGCGAGCTCAGGTACTTCTG - 3'(Reverse)(SEQ ID NO: 4) |
| IL-6 | 5'-AGGGCTCTTCGGCAAATGTA- 3'(Forward)(SEQ ID NO: 5) | 5'- GAAGGAATGCCCATTAACAACAA- 3'(Reverse)(SEQ ID NO: 6) |
| GAPDH | 5'-GCCACATCGCTCAGACACC- 3'(Forward)(SEQ ID NO: 7) | 5'-CCCAATACGACCAAATCCGT- 3'(Reverse)(SEQ ID NO: 8) |

### 1-9. Bronchoalveolar Lavage Fluid Analysis

Bronchoalveolar lavage fluid (BALF) was obtained after anesthetizing each mouse with intranasal isoflurane. After tracheotomy was performed to expose the trachea, a catheter was inserted, and 1 mL of PBS was slowly injected and collected through the catheter, and the procedure was repeated three times. Thereafter, the recovered BALF was centrifuged at 1,000 g for 10 minutes at 4°C, and the precipitated cells were resuspended in 500 µL of PBS. The cells were stained with the Diff-Quik kit (Sysmex, Kobe, Japan), observed under a microscope, and sorted, and then the total cells and neutrophils were counted.

### 1-10. Lung Wet/Dry Ratio Measurement

To determine the severity of pulmonary edema, the wet weight of the right upper lung was measured, the tissue was dried 60°C for 72 hours, and the dry weight was measured. Afterwards, the W/D ratio was calculated.

### 1-11. Statistical Analysis

Statistical analysis of the results was performed using GraphPad Prism 5 software (GraphPad Software, San Diego, CA). All data are expressed as mean ± S.E.M. Statistical analysis of cell test data was performed by an unpaired t-test (one-tailed), and statistical analysis of animal test data was performed by ANOVA Tukey analysis. Here, p values less than 0.05 were considered statistically significant.

### Example 2. Cytotoxicity Assay of Gancaonin N

To examine the cytotoxicity of gancaonin N, the inventors treated Raw264.7 cells and A549 cells with gancaonin N according to the methods of Examples 1 to 4 and then measured cell viability.

As a result, as shown in FIG. 1A (Raw264.7 cells) and FIG. 1B (A549 cells), it was found that the cell viability did not decrease when the cells were treated with gancaonin N as compared to the control group. On the basis of this fact, it was found that gancaonin N induced no cytotoxicity regardless of the concentration of gancaonin N.

### Example 3 Investigation of NO Production Inhibitory Effect of Gancaonin N

To investigate the anti-inflammatory effect of gancaonin N, the inventors pretreated RAW264.7 cells with gancaonin N, 2 hours before stimulation with LPS, and measured the amount of NO production as described in Examples 1 to 5 above to determine change in the amount of NO production according to the concentration of gancaonin N used to treat the cells.

As a result, as shown in FIG. 2A, it was confirmed that the production of NO significantly decreased as the treatment concentration of gancaonin N increased. From this result, it was confirmed that gancaonin N had the effect of inhibiting the production of NO.

### Example 4. Investigation of Inhibitory Effect of Gancaonin N on Inflammation-related Factors

To investigate the anti-inflammatory effect of gancaonin N, the inventors treated Raw264.7 cells with LPS and Gancaonin N, as described in Examples 1 to 6, and evaluated the expression level of inflammation-inducing factors. Thus, the inhibitory effect of gancaonin N on inflammation-related factors was specifically confirmed. As a result, it was confirmed that the expression levels of proteins of PGE2 (FIG. 2B), iNOS (FIG. 2C), and COX-2 (FIG. 2D) significantly decreased as the treatment concentration of gancaonin N increased. From this result, it was specifically confirmed that the gancaonin N of the present disclosure can significantly inhibit the protein expression of PGE2, iNOS, and COX-2 related to the inflammatory response.

### Example 5. Investigation of Inhibitory Effect of Gancaonin N on Expression of Pro-inflammatory Cytokine

### 5-1. Checking of mRNA Expression Level of Pro-inflammatory Cytokine

To investigate the inhibitory effect of gancaonin on the expression levels of pro-inflammatory cytokines, the mRNA expression levels of tumor necrosis factor-a (hereinafter referred to as TNF-α), interleukin-1 beta (hereinafter referred to as IL-1β), and interleukin 6 (hereinafter referred to as IL-6) which are all known as pro-inflammatory cytokines were measured. Specifically, A549 cells were treated with LPS and gancaonin N as described in Examples 1 to 8, and the mRNA expression levels were checked.

As a result, as shown in FIGS. 3A to 3C, when the cells were treated with gancaonin N, it was confirmed that the mRNA expression of the pro-inflammatory factors including TNF-α (FIG. 3A), IL-1β (FIG. 3B) and IL-6 (FIG. 3C) was significantly inhibited. It was also confirmed that the inhibitory effect was proportional to the amount of gancaonin N used for treatment.

### 5-2. Checking of Protein Expression Level of Pro-inflammatory Cytokine

For the pro-inflammatory cytokines whose mRNA expression inhibition was confirmed as above, the expression level of the protein encoded by the mRNA was further checked. To determine the protein expression level, A549 cells were treated with LPS and gancaonin N as described in Examples 1 to 8, and the protein expression levels were checked.

As a result, as shown in FIGS. 4A to 4C, when the cells were treated with gancaonin N, it was confirmed that the protein expression of the pro-inflammatory factors TNF-α (FIG. 4A), IL-1β (FIG. 4B) and IL-6 (FIG. 4C) was significantly inhibited. In the case of the expression of COX-2, which is a factor related to inflammation, it was also confirmed that as shown in FIG. 4D, the protein expression of COX-2 was significantly inhibited.

On the basis of the results described above, the inventors have determined that when cells are treated with gancaonin N, both the mRNA expression of pro-inflammatory cytokines and the expression of proteins encoded by the mRNA can be significantly inhibited, and the inhibitory effect is proportional to the amount of gancaonin N used for the treatment.

### Example 6. Investigation of Role of Gancaonin N in MAPK/NF-κB Mechanism

Through the Examples 3 to 5 described above, it was confirmed that gancaonin N significantly inhibited inflammation-related factors. To identify through which pathway the anti-inflammatory response occurs, the role of gancaonin N in the MAPK/NF-κB signaling pathway was investigated. To this end, A549 cells were treated with different amounts of gancaonin N (5µM to 40 µM) 2 hours before LPS treatment, and then, as described in Examples 1 to 7, the protein expression levels and activity levels of extracellular signal-regulated kinase (ERK), p38 and p65, which are factors associated with the MAPK/NF-κB signaling pathway were checked.

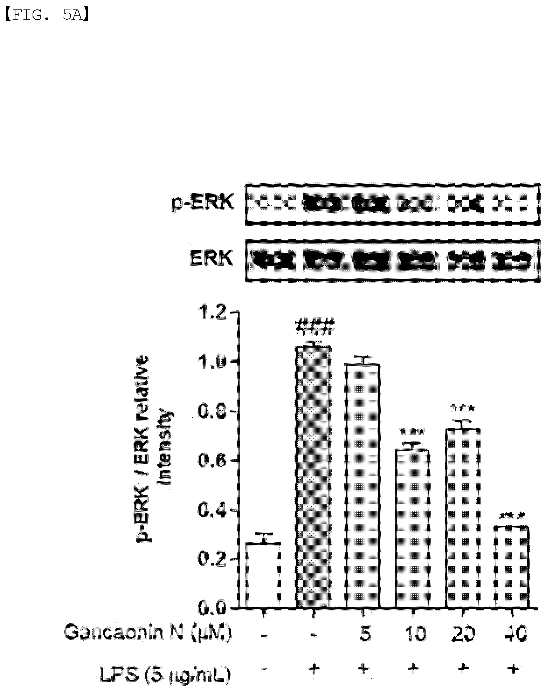

As a result, as shown in FIGS. 5A to 5C, it was confirmed that the activity/protein expression levels of ERK (FIG. 5A), p38 (FIG. 5B) and p65 (FIG. 5C) were significantly reduced when treated with gancaonin N. More specifically, it was confirmed that the inhibitory effect on the activity/protein expression levels of ERK and p65 was most excellent when the amount of gancaonin N used was 40 µM, and the inhibitory effect on the activity/protein expression levels of p38 was most excellent when the amount of gancaonin N used as 20 µM. The result of taking images of p65 whose expression level was confirmed through immunofluorescence is shown in FIG. 5D.

### Example 7. Investigation of Anti-inflammatory Effect of Gancaonin N in Lung Injury Mouse Model

The mouse models prepared as described in Examples 1 to 3 were treated with LPS to induce acute lung injury (ALI), and then the anti-inflammatory effect of gancaonin N was specifically evaluated for the mice of five groups (control, vehicle (only LPS), gancaonin N (10 mg/kg), gancaonin N (30 mg/kg), and dexamethasone (3 mg/kg)),

### 7-1. Investigation of Effect of Gancaonin N on Inflammatory Cell Infiltration

To specifically investigate the infiltration effect of inflammatory cells, gancaonin N was injected intraperitoneally into the mice before intratracheal administration of LPS to the mice as described in Examples 1 to 9, and the bronchoalveolar lavage fluid (BALF) for each mouse was stained with the Diff-Quik kit for the preparation of analysis. The staining results are shown in FIG. 6A, and the cell count and the neutrophil count are shown in FIGS. 6B and 6C, respectively. For each case where treatment with gancaonin N was performed, the counts were significantly reduced. When treated with gancaonin N at 30 mg/kg, the number of cells and neutrophils were similar to those of the group treated with dexamethasone (DEX), which is conventionally used as an anti-inflammatory drug.

### 7-2. Investigation of Relationship between Severity of Pulmonary Edema and Gancaonin N

To investigate the ameliorative effect of gancaonin N on the severity of pulmonary edema, after treatment with gancaonin N, the wet and dry rung ratio (W/D ratio) of pulmonary edema was checked. As a result, as shown in FIG. 7, when treated with gancaonin N, it was confirmed that the W/D ratio was significantly lowered. Specifically, the group treated with 30 mg/kg of gancaonin N showed a W/D ratio similar to that of the group treated with dexamethasone.

### 7-3. Investigation of Inhibitory Effect of Gancaonin N on Pro-inflammatory Factor

To specifically investigate whether gancaonin N can inhibit pro-inflammatory factors in a mouse model, the protein expression levels of COX-2, IL-1β, and TNF-α were measured by Western blotting as described in Examples 1 to 6 above. As a result, it was confirmed when treated with gancaonin N, the expression of the pro-inflammatory factors was significantly reduced. That is, it was confirmed that the group treated with 30 mg/kg of gancaonin N exhibited similar protein expression levels of the pro-inflammatory factors to those of the group treated with dexamethasone.

On the basis of the above results of specific experiments, the inventors confirmed that gancaonin N could inhibit the expression levels of anti-inflammatory factors *in vitro* and *in vivo.* Therefore, it can be concluded that gancaonin N can be usefully used for the treatment of inflammatory diseases and especially diseases related to inflammation of the lung.

### Industrial Applicability

The pharmaceutical composition according to the present disclosure, which contains as an active ingredient gancaonin N, which is a flavonoid component derived from the root and aerial parts of licorice (Glycyrrhiza aspera Pall), is confirmed to inhibit the production of nitric oxide (NO), which is a key factor of inducing inflammation, to inhibit the expression of PGE2, iNOS and COX-2, which are known as factors of inducing inflammation, and not to exhibit cytotoxicity. Therefore, the composition containing gancaonin N as an active ingredient, which is proposed by the present disclosure, can be usefully used as a safe natural therapeutic agent with few side effects for inflammatory diseases and more specifically lung inflammation.

## Claims

1. A pharmaceutical composition for use in a method for preventing or treating an inflammatory disease which is pneumonia, the composition comprising gancaonin N as an active ingredient.

2. The composition for use of claim 1, inhibiting production of at least one selected from the group consisting of nitric oxide (NO), prostaglandin 2 (PGE2), and cyclo oxygenase-2 (COX-2).

3. The composition for use of claim 1, inhibiting expression of pro-inflammatory cytokines and expression of genes encoding the pro-inflammatory cytokines.

4. The composition for use of claim 3, wherein the pro-inflammatory cytokine comprises any one or more selected from the group consisting of tumor necrosis factor-a (TNF-α), interleukin-1β (IL-1β), and interleukin 6 (IL-6).

5. The composition for use of claim 1, inhibiting expression of at least one selected from the group consisting of extracellular signal-regulated kinase (ERK), p38, and p65.

6. The composition for use of claim 1, wherein the gancaonin N is comprised in an amount that falls within a range of 5 µM to 60 µM.

7. A health functional food composition for use in a method for preventing or treating an inflammatory disease which is pneumonia, the composition comprising gancaonin N as an active ingredient.

## Patentansprüche

1. Pharmazeutische Zusammensetzung zur Verwendung in einem Verfahren zur Vorbeugung oder Behandlung einer entzündlichen Erkrankung, bei der es sich um Pneumonie handelt, wobei die Zusammensetzung Gancaonin N als Wirkstoff umfasst.

2. Zusammensetzung zur Verwendung nach Anspruch 1, wobei die Produktion von mindestens einem gehemmt wird, das ausgewählt ist aus der Gruppe bestehend aus Stickstoffmonoxid (NO), Prostaglandin 2 (PGE2) und Cyclooxygenase-2 (COX-2).

3. Zusammensetzung zur Verwendung nach Anspruch 1, wobei die Expression von proinflammatorischen Zytokinen und die Expression von Genen, welche die proinflammatorischen Zytokine codieren, gehemmt wird.

4. Zusammensetzung zur Verwendung nach Anspruch 3, wobei die proinflammatorischen Zytokine eines oder mehrere umfassen, ausgewählt aus der Gruppe bestehend aus Tumornekrosefaktor-α (TNF-α), Interleukin-1β (IL-1β) und Interleukin 6 (IL-6).

5. Zusammensetzung zur Verwendung nach Anspruch 1, wobei die Expression von mindestens einem gehemmt wird, das ausgewählt ist aus der Gruppe bestehend aus extrazellulär signalregulierter Kinase (ERK), p38 und p65.

6. Zusammensetzung zur Verwendung nach Anspruch 1, wobei das Gancaonin N in einer Menge umfasst ist, die in einem Bereich von 5 µM bis 60 µM liegt.

7. Funktionelle Lebensmittelzusammensetzung zur Gesundheitsförderung zur Verwendung in einem Verfahren zur Vorbeugung oder Behandlung einer entzündlichen Erkrankung, bei der es sich um Pneumonie handelt, wobei die Zusammensetzung Gancaonin N als Wirkstoff umfasst.

## Revendications

1. Composition pharmaceutique destinée à être utilisée dans un procédé de prévention ou de traitement d'une maladie inflammatoire, à savoir la pneumonie, dans lequel la composition comporte la gancaonine N en tant que principe actif.

2. Composition destinée à être utilisée selon la revendication 1, inhibant la production d'au moins un élément choisi parmi le groupe comprenant l'oxyde nitrique (NO), la prostaglandine 2 (PEG2) et la cyclo-oxygénase-2 (COX-2).

3. Composition destinée à être utilisée selon la revendication 1, inhibant l'expression des cytokines pro-inflammatoires et l'expression des gènes codant pour les cytokines pro-inflammatoires.

4. Composition destinée à être utilisée selon la revendication 3, dans lequel la cytokine pro-inflammatoire comporte une ou plusieurs substances choisies parmi le groupe constitué du facteur de nécrose tumorale α (TNF- α) de l'interleukine-1β (IL-1β) et de l'interleukine 6 (IL-6).

5. Composition destinée à être utilisée selon la revendication 1, inhibant l'expression d'au moins un élément choisi parmi le groupe comprenant la kinase régulée par des signaux extracellulaires (ERK), p38 et p65.

6. Composition destinée à être utilisée selon la revendication 1, dans lequel la gancaonine N est présente en une quantité comprise dans une plage de 5 µM à 60 µM.

7. Composition d'aliments fonctionnels pour la santé destinée à être utilisée dans un procédé de prévention ou de traitement d'une maladie inflammatoire, à savoir la pneumonie, dans lequel la composition comporte la gancaonine N en tant que principe actif.
